# EUROPEAN PATENT APPLICATION

(11) **EP 3 628 230 A1**
(43) Date of publication of application: **01.04.2020**
(21) Application number: 18197207.6
(22) Date of filing: 27.09.2018
(51) Int. Cl.: A61B 6/03, A61B 6/12, A61B 6/00, G06T 7/00, G06T 11/00, G06T 7/73

(54) **X-RAY IMAGING SYSTEM WITH FOREIGN OBJECT REDUCTION**

(71) Applicant: Koninklijke Philips N.V., 5656 AG Eindhoven (NL)
(72) Inventor: VUPPALA, Sunil Kumar, 5656 AE Eindhoven (NL); BHAT, Ravindra, 5656 AE Eindhoven (NL); VOGTMEIER, Gereon, 5656 AE Eindhoven (NL); SISODIA, Rajendra Singh, 5656 AE Eindhoven (NL); CHAUDHURY, Sudipta, 5656 AE Eindhoven (NL)
(74) Representative: de Haan, Poul Erik

(57) **Abstract**

An improved X-ray imaging system. The system comprises an X-Ray radiation source configured to emit an X-ray beam towards an object to be imaged and an X-ray detector configured to detect X-rays which have passed through the object. It further comprises a reconstruction processing means configured to reconstruct an image of the object based on the detected X-rays, wherein the reconstruction processing means is further configured to determining presence of at least one foreign object located in the object or located between a surface of the object and the X-Ray radiation source and/or the X-ray detector, obtaining a three-dimensional profile of the determined foreign object from a server-based foreign object database, and reconstructing an image of the object by acquiring a plurality of projection images of the object from the X-ray detector and reducing impact on image quality of an artefact that can be caused by the foreign object in the image of the object based on at least the obtained three-dimensional profile of the foreign object.

## Description

### FIELD OF THE INVENTION

The invention relates to an X-ray imaging system, to a method of imaging an object by an X-ray imaging system, and to a computer program element.

### BACKGROUND OF THE INVENTION

X-ray imaging may be used to provide anatomical information, in particular in diagnostic and/or therapeutic applications. In particular in computer tomography (CT) and magnetic resonance imaging (MRI) systems, 3D data reconstruction of acquired cross-sectional images can be combined into a three-dimensional image of an object and may provide a three-dimensional volume information.

During imaging, the presence of metals and/or other materials of foreign objects may disturb the reconstruction and may lead to severe artefacts - often streak artefacts - that may have influence on the image information and degrade an image quality, and thus the diagnostic quality. Such foreign object may be, for example, implants of a patient to be imaged. For example, such foreign objects may be dental implants, bone screws or the like.

There exist a range of artefact reduction solutions for X-ray imaging. Some of these are based on algorithmically identifying a three-dimensional volume of the foreign object in the reconstructed image of the object and require subsequently rerun the reconstruction considering the identified three-dimensional volume of the foreign object.

Such approaches may not only be computationally intensive, but may also be limited to comparatively large foreign objects of high density.

### SUMMARY OF THE INVENTION

There may therefore be a need to improve imaging, in particular regarding image quality in connection with foreign objects.

The object of the present invention is solved by the subject matter of the independent claims where further embodiments are incorporated in the dependent claims. It should be noted that the following described aspect of the invention equally applies to the X-ray imaging system, to the method of imaging an object by an X-ray imaging system, and to the computer program element.

According to one aspect there is provided an X-ray imaging system, comprising:
an X-Ray radiation source configured to emit an X-ray beam towards an object to be imaged,
an X-ray detector configured to detect X-rays which have passed through the object,
a reconstruction processing means configured to reconstruct an image of the object based on the detected X-rays, wherein the reconstruction processing means is further configured to:
   determining presence of at least one foreign object located in the object or located between a surface of the object and the X-Ray radiation source and/or the X-ray detector,
   obtaining a three-dimensional profile of the determined foreign object from a server-based foreign object database, and
   reconstructing an image of the object by acquiring a plurality of projection images of the object from the X-ray detector and reducing impact on image quality of an artefact that can be caused by the foreign object in the image of the object based on at least the obtained three-dimensional profile of the foreign object.

The X-ray imaging system may be configured for CT, MRI or other X-ray applications for imaging an object where artefacts may be caused due to foreign objects. In medical imaging the object may also be referred to as a patient, wherein the foreign object may be located inside the patient, e.g. as an implant, or may be located outside the patient, for example as an electronic or metallic component, like a sensor, a patient monitoring means, a patient fixation means, or the like.

The reconstruction processing means may comprise one or more processors, a memory for storing at least one program element, a memory for storing a series of cross-sectional images of the object and/or a memory for storing a reconstructed three-dimensional image etc., a data interface etc., and a reconstruction software module or the like. The reconstruction processing means may be configured to calculate, for example, an X-ray absorption effect or the like of the foreign object and to provide respective corrected data for a reconstruction algorithm or the like without truncation.

Obtaining the three-dimensional profile of the determined foreign object from the foreign object database may also be referred to as downloading the same from a respective server, which may be a local computer system, a cloud computing system or the like. The three-dimensional profile may be provided by a manufacturer of the foreign object and/or may be determined, for example, by a scan by means of the X-ray system. It may comprise images, in particular three-dimensional images, preferably X-ray images, of the foreign object. In other words, the determined foreign object may have been measured and/or analyzed before and may be recorded to the foreign object database and/or such data may be provided by a supplier of the foreign object to have, for example, an exact material and/or geometry model of the foreign object that can be used for the improvement of the reconstruction.

Reducing impact on image quality of the foreign object may be performed directly during a process of reconstruction and may be understood as at least partly avoiding the artefact to be apparent in the image of the object, and/or as at least partly removing the artefact from the image etc.

The X-ray imaging system may improve image correction in particular in terms of artefact reduction and hence improve image quality. In particular, correction of the artefact that may be caused by the foreign objected located in the field-of-view between the X-ray radiation source and the X-ray detector may be improved. The three-dimensional profile of the foreign object may be provided with a high level of detail improving the overall accuracy of correcting the image of the object. By way of example, the system may improve clarity of the image of the object. It may resolve small foreign objects from the object image and/or the acquired images. It may reduce artefacts of low density thin walled metals.

In an example, the reconstruction processing means may further be configured to at least partly subtract the artefact caused by the foreign object from the image of the object for reducing the impact of the same on image quality. The reconstruction processing means may comprise a subtraction module configured to subtract at least a fraction of the artefact of the foreign object. The subtraction module may, for example, be implemented as a software module. The artefact may be included in on or more of the acquired images.

In a further example, the three-dimensional profile may comprise density data and/or material data of the foreign object. The density data may change in the direction of imaging or relative thereto. The material data may comprise a single material or a composite material. The density data may comprise a density map, in particular a multi-dimensional density map. Thus, more information about the foreign object, which may further improve image quality.

In another example, the three-dimensional profile and/or comprises a scatter profile of the foreign object. Optionally, the scatter profile may comprise different intensities. The scatter profile may be determined prior to acquiring the images of the object. The reconstruction processing means may further be configured to reduce the artefact based on the obtained scatter profile of the foreign object. Thus, more information about the foreign object, which may further improve image quality.

In an example, in the foreign object database an identifier may be associated with the three-dimensional profile, wherein the reconstruction processing means may further be configured to determine the identifier of the foreign object to obtain the three-dimensional profile data from the foreign object database.

In a further example, the reconstruction processing means may further be configured to determine the identifier from the one or more acquired images of the object including the foreign object. Thus, the foreign object may already be identified on basis of the acquired images.

In another example, the reconstruction processing means comprises a classification means for identifying the foreign object on basis of at least a feature extraction, which may reveal a shape, material property, density or the like, of the foreign object derived from the one or more acquired images of the object which include the foreign object. The classification means may be implemented as a software module, in particular an artificial-intelligence-module, AI-module. It may comprise a machine learning component implemented in an artificial neural network, in particular a convolutional neural network, or may be arranged instead as a support vector machine, linear regression algorithm or otherwise. The classification means may be pre-trained with suitable training data sets. Further, it may be configured to compare the identified foreign object with records of the foreign object database to automatically obtain the correct three-dimensional profile and, optionally, further information, like e.g. material properties, density distribution or the like, for reducing the impact of the artefact caused by the foreign object.

In an example, X-ray absorption measurements from the image data may be used to give a first analysis of the material and/or a spatial distribution of the material of the foreign object. Such an information may be used to perform or assist classification of the foreign object.

In a further example, the reconstruction processing means may further be configured to identify the foreign object based on non-shape related properties of the foreign object derived from the acquired image and matched with the foreign object database. This may be used as the single identification method or may be combined with the identifying method of the classification means to verify classification and/or improve matching the foreign object in the corresponding database.

In an example, the reconstruction processing means is further configured to identify the foreign object based on a read-out of a computer-readable identifier provided along with the foreign object. The identifier may be unique and may be provided as a barcode, a QR code, an RFID-tag, an NFC tag or the like that can read by a barcode-scanner, a camera, a radio module etc. of the X-ray imaging system. If the foreign object is an implant, it may be identified using such a code or information obtained by a patient information system. This may be used as the single identification method or may be combined with the identifying method of the classification means to verify classifying and/or improve matching the foreign object in the corresponding database.

In a further example, the reconstruction processing means may further be configured to determine at least a 6DoF-position (six degrees of freedom-position), optionally instead or in addition also an orientation, of the foreign object relative to the object before reducing the impact of the foreign object. The information on position and/or orientation of the foreign object relative may, for example, may be determined based on one or more of the acquired images of the object including the foreign object, in particular also before or prior to reconstruction. For this, a few orthogonal images may be acquired to reduce computational needs or, a low resolution three-dimensional image may be generated, e.g. reconstructed, to reduce computational needs and increase speed. Or a fully conducted reconstruction is performed without artefact reduction, e.g. artefact subtraction.

In an example, the X-ray imaging system, e.g. the reconstruction processing means or a separate module, may be configured to acquire 6DOF position and/or orientation of the foreign object using one or more of the following methods: electromagnetic tracking, radar and/or light and/or ultrasound based triangulation. In addition or alternatively, an acceleration sensor, a magnetic sensor or the like, may provide additional 6DOF information.

In a further example, the reconstruction processing means may further be configured to determine the 6DoF-position, and optionally its orientation, based on a first image acquiring scan provided with a first resolution, and based on the 6DoF-position to subtract the foreign object from a second image acquiring scan provided with a second resolution higher than the first resolution. The first image acquiring scan may also be referred to as a scout scan, which may be faster than the second scan. Due to determining 6DoF-position and/or orientation based on the scout scan a definition of quality and position may lead to a patient and/or scanner guiding information. After processing the scout scan and deriving the information therefrom, the second, subsequent or final high resolution diagnostic scan may be performed automatically.

Generally, data obtained from such a scout scan may be used to determine the identifier of the foreign object. Based on the determined identifier, the corresponding data of the foreign data base may be obtained.

In another example, the reconstruction processing means may further be configured to adapt scan parameters of the X-Ray imaging system based on the 6DoF-position. The scan parameters may include at least one of a patient position, a tilt angle, a start and an end of the scan, etc.

In an example, the foreign object may comprise an electronic device arranged between a surface of the object and the X-Ray radiation source and/or the X-ray detector. The electronic device may be a sensor, camera or the like. Thus, foreign objects located outside the object may be determined and the image of the object may be corrected accordingly.

In a further example, the foreign object may comprise at least one part of a patient monitoring system. The patient monitoring system may be a sensor, camera or the like. Alternatively or in addition, the patient monitoring system may comprise fiducial markers or the like.

In another example, the foreign object may comprise a patient fixation means.

In a further example, the foreign object may be an implant located inside the object to be imaged. Such an implant may be patient specific, e.g. a titanium hip or the like, and may generate a metal artefact. The three-dimensional model may comprise at least geometry and/or material data, e.g. density information etc., of the implant. In some embodiments the patient implant may be manufactured with additive manufacturing / 3D printing.

According to an aspect, provided is a method of imaging an object by an X-ray imaging system, comprising the steps of:
determining presence of at least one foreign object located in the object or located between a surface of the object and the X-Ray radiation source and/or the X-ray detector,
obtaining a three-dimensional profile of the determined foreign object from a server-based foreign object database, and
reconstructing an image of the object by acquiring a plurality of projection images of the object from the X-ray detector and reducing impact on image quality of an artefact that can be caused by the foreign object in the image of the object based on at least the obtained three-dimensional profile of the foreign object.

The method may be performed with the system described above. In some embodiments, for example, it may be stored as a program element on a computer-readable medium, which, when being executed by a processor (for example a processor of the X-ray system, in particular of the reconstruction processing means) is adapted to carry out the steps of the method as described in the above and in the following.

In an example, the three-dimensional profile may be obtained from at least a first scan of the foreign object on a first radiation dosage level and a second scan on a second radiation dosage level different to the first radiation dosage level of the X-ray imaging system. Alternatively or in addition, the thee-dimensional profile of the foreign object may be obtained by scanning the foreign object at multiple energy settings, in particular multiple scans at different kV settings. Information of the different radiation dosage levels and/or the multiple energy setting may be stored as additional information in the foreign object database.

In a further example, a photon counting based scatter profile of the foreign object may be determined before reconstructing the image from the projection images. Photon counting based scatter profile may be used during image reconstruction for further improving accuracy of the artefact and/or streak reduction, e.g. removal, subtraction etc. The scatter profile may be used in combination with the three-dimensional profile of the foreign object, which may improve image quality, in particular improve accuracy of subtracting the artefact from the image of the object. For example, using the scatter profile may improve image correction of small metal artefacts.

In another example, a phase contrast information of the foreign object is determined before reconstructing the image. In other words, dark field x-ray imaging, which is based on a phase contrast information, may be used in combination with the three-dimensional profile of the foreign object and/or the scatter profile. This may further improve image correction and hence further improve image quality.

In an example, the three-dimensional profile of the foreign object may be obtained by scanning the foreign object using the X-ray imaging system. Thereby, the three-dimensional profile of the foreign object may be corrected based on the scatter profile, the dark field x-ray imaging, or a combination thereof. This may further improve image correction and hence improve image quality.

In a further example, determining the presence of the foreign object may be performed during a scout scan provided with a first radiation dose, and acquiring the image of the object may be performed by an examination scan provided with a second radiation dose higher than the first radiation dose. After determining the presence of the foreign object, it may be identified as explained above. For example, the foreign object may be classified using the above classification means or the like. The scout scan may be may take less time and may provide patient and/or scanner guiding information. The examination scan may be performed automatically when the foreign object could be identified.

In an example, the three-dimensional profile is at least partly obtained by additive manufacturing, e.g. 3D printing, a model of the determined foreign object and scanning the same by means of the X-ray imaging system. For example, the foreign object may be determined manually before scanning, i.e. performing the scout scan and/or the examination scan. In some embodiments, the foreign object may be determined automatically as explained above. The manufactured model may be scanned to obtain the scatter profile, the density information etc.

According to an aspect a computer program element is provided, which, when being executed by at least one processing unit, e.g. a processor of the X-ray imaging system, in particular of the reconstruction processing means, is adapted to cause the processing unit to perform the method as explained above. A computer-readable medium may be a floppy disk, a hard disk, an USB (Universal Serial Bus) storage device, a RAM (Random Access Memory), a ROM (Read Only memory) and an EPROM (Erasable Programmable Read Only Memory). A computer readable medium may also be a data communication network, e. g. the Internet, which allows downloading a program code.

A further aspect of the invention relates to a program element (for example a computer program) for controlling an X-ray system which, when being executed by a processor (for example a processor of the X-ray imaging system), is adapted to carry out the steps of the method as described in the above and in the following.

These and other aspects of the present invention will become apparent from and elucidated with reference to the embodiments described hereinafter.

### BRIEF DESCRIPTION OF THE DRAWINGS

Exemplary embodiments of the invention will be described in the following with reference to the following drawings:
Fig 1 shows schematically an X-ray system according to an exemplary embodiment of the invention in a perspective view.
Fig. 2 shows schematically a further embodiment of an X-ray imaging system in a side view.
Fig. 3 shows schematically a block diagram of an exemplary operation of an X-ray imaging system.
Fig. 4 shows schematically a block diagram of another exemplary operation of an X-ray imaging system.
Fig. 5 shows schematically images of an object to be imaged at several points during a reconstruction and/or correction process.
Fig. 6 shows a flow chart of a method of imaging an object by an X-ray imaging system.

The figures are merely schematic representations and serve only to illustrate embodiments of the invention. Identical or equivalent elements are in principle provided with the same reference signs.

### DETAILED DESCRIPTION OF EMBODIMENTS

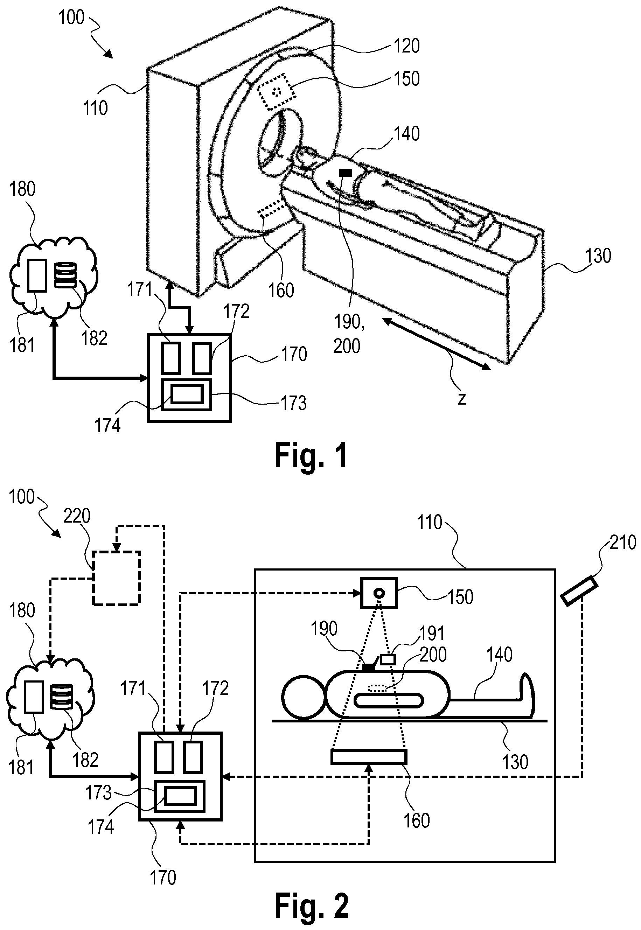

Figure 1 shows schematically an X-ray imaging system 100, which is in this embodiment a computed tomography imaging scanner. The X-ray imaging system 100 comprises a stationary housing 110 and a rotatable gantry 120 which is rotatable over an angular range of about 360° about an object support 130, which is in this embodiment a support table. In this embodiment, an object 140 to be imaged, which is exemplarily a human patient, is located on an upper surface of the object support 130. The object support 130 is at least translationally movable along a z-axis to selectively move the object 140 into the housing 110. It is noted that the object support 130 may have 6DoF (six degrees of freedom) in some embodiments. The X-ray imaging system 100 further comprises a radiation source 150 that is configured to emit an X-ray radiation beam towards the object 140 to be imaged, and in particular configured to generate the radiation beam to be directed into an examination region. The radiation beam interacts with a region of interest of the object 140 disposed in the examination region (see Figure 2), wherein spatially varying absorption of the radiation is generated, as it passes through the examination region.

The X-ray imaging system 100 further comprises an X-ray detector 160 configured to detect X-rays which have passed through the object 140 and in particular configured to detect an absorption-attenuated radiation after having passed through the examination region. In this embodiment, the radiation source 150 and the X-ray detector 160 are mounted to the gantry 120 and are arranged opposite each other, so that the X-ray detector 160 continuously receives X-rays from the radiation source 150. The X-ray detector 160 may comprise a two-dimensional array of detector elements, wherein other embodiments may be contemplated.

The X-ray imaging system 100 further comprises one or more computational means, wherein in this embodiment mainly a reconstruction processing means 170 will be described. The reconstruction processing means 170 is connected to at least the X-ray detector 160 and/or the radiation source 150 to control these and/or to obtain data therefrom, in particular from the X-ray detector 160. The reconstruction processing means 170 may also be formed by several subsystems, function modules or units, software modules or units, or the like (not further detailed here), and is configured to reconstruct an image of the object 140 based on the X-rays detected by the X-ray detector 160, and in particular based on a plurality of acquired projection images of the object 140. In this embodiment, the reconstruction processing means 170 comprises at least one processor 171, such as a backprojection processor, or the like, at least one memory 172 for storing image data and at least one memory 173 for storing one or more program elements. The reconstruction processing means 170 comprises at least one image reconstruction algorithm which, in this embodiment, is stored as a program element in the memory 173. Reconstruction processing means 170 is, in this embodiment, configured to reconstruct the image using a filtered backprojection. However, also other reconstruction algorithms can be used for reconstructing.

The reconstruction processing means 170 further comprises an artificial intelligence module, AI-module, which for better illustration is denoted by reference sign 174. The AI-module 174 comprises a classification means, which is configured to identify objects within the images, in particular within the images reconstructed by the reconstruction processing means 170. The classification means of the AI-module 174 exemplarily comprises a machine learning component implemented in an artificial neural network, in particular a convolutional neural network, which may be arranged instead as a support vector machine, linear regression algorithm or otherwise. The classification means may be pre-trained with suitable training data sets.

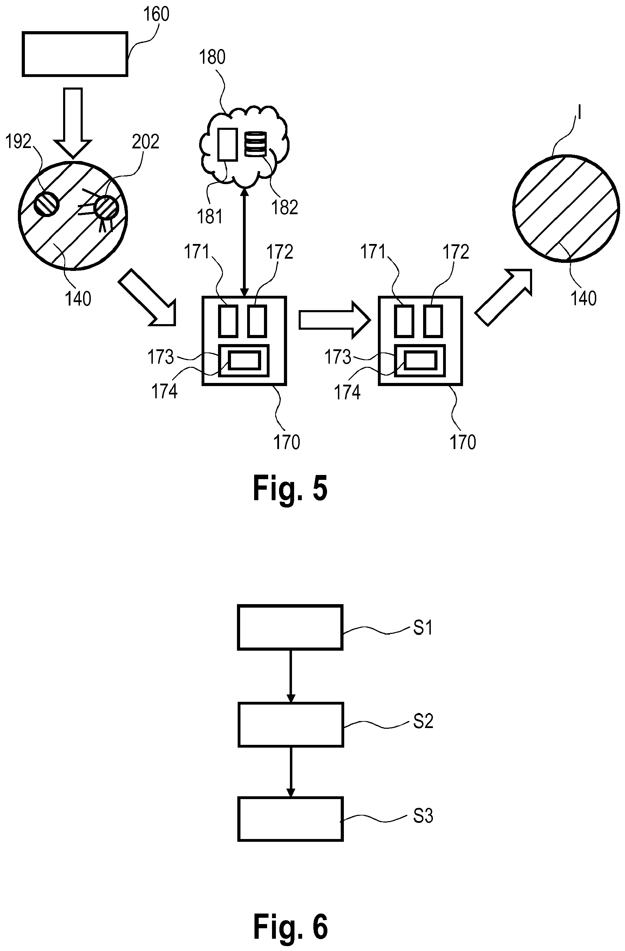

The X-ray imaging system 100 further comprises a server-based computing device 180, which may be a local or, as exemplarily indicated in Figure 1, a cloud computing system. The server-based computing device 180 is connected to at least the reconstruction processing means 170, wherein this connection may be established by wire or wireless. The server-based computing device 180 comprises a processor, memory etc. which are collectively designated by reference sign 181. The server-based computing device 180 further comprises a database 182 configured to associate an identifier of a foreign object 190, 200, which will be described in more detail below, with a corresponding three-dimensional profile of the foreign object 190, 200. The foreign object database 182 comprises the three-dimensional profile, geometric data, such as shape and/or dimensional data, material data, density data, density contribution data, X-ray absorption data, a scattering profile, or the like. These data may be measured and/or analyzed prior during a scout or examination scan of the X-ray imaging system 100 or may be provided by a manufacturer and/or supplier of the foreign object 190, 200. In this embodiment, a first foreign object 190 is located outside the object 140 between an outer surface of the object 140 and the radiation source 140. Byway of example, the first foreign object 190 is an electronic device, such as sensor, a camera, or the like. Further exemplarily, a second foreign object 200 is located inside the object 140 and is formed, for example, as a metal implant, such as a metal clip, a high-density dental filling, an artificial hip, or the like. It is noted that the first and second foreign object 190, 200 may cause artefacts, in particular if the imaged region of interest contains their high density regions. Typically, the second foreign object 190 may appear as a metal artefact in a reconstructed image as streaks emanating from the high density region (see Figure 5).

Referring to Figure 2, which schematically shows a further embodiment of the X-ray imaging system 100 without occlusion through the housing 110, the first foreign object 190, which is located outside the object 140, exemplarily further comprises a computer-readable identifier 191 that is provided along with the first foreign object 190. It is noted that the size of the computer-readable identifier 191 is only exaggerated for better illustration. The computer-readable identifier 191 maybe provided as an electronic or-non electronic device, such as barcode, QR code, NFC tag, RFID tag, or the like, or may comprise an accelerometer, a magnetic sensor, or the like, and may further be configured to be read-out by a detection means 210, as schematically indicated in Figure 2. Depending on the design of the identifier 191, the detection means 210 may be provided as a barcode or QR code scanner, a NFC module, a radio module, a camera, or the like. It is noted that the detection means 210 may be arranged outside or inside the housing 110. In this embodiment, the detection means 210 is connected to the reconstruction processing means 170. It is noted that the detection means 210 may also be connected directly to the server-based computing device 180. The same or a further detection means 210 may be configured to detect the foreign object 190 based on EM tracking, radar based triangulation, light based triangulation, ultrasound based triangulation, or the like.

Further referring to Figure 2, in this embodiment, the X-ray imaging system 100 further comprises an additive manufacturing device 220, which is configured to form a physical model, e.g. by 3D printing technology, of the foreign object 190, 200 imaged by the X-ray imaging system 100. By way of example, the additive manufacturing device 220 is connected on one side to the reconstruction processing means 170 and on another side to the server-based computing device 180. The additive manufacturing device 220 is configured to generate a 3D model of the foreign object 190, 200 based on a foreign object image reconstructed by the reconstruction processing means 170. Such a 3D model of the foreign object 190, 200 may be imaged by the X-ray imaging system 100 and provided to server-based computing device 180.

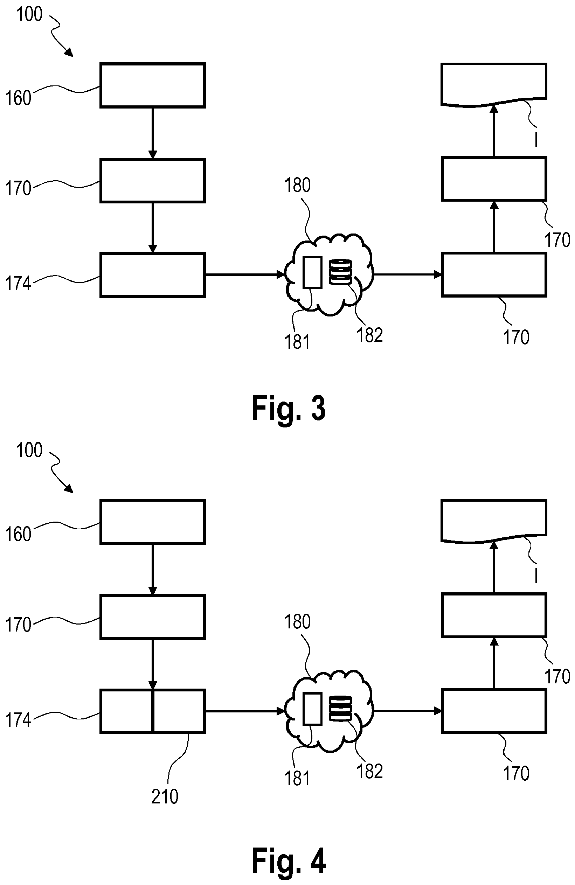

Figure 3 shows a schematic block diagram of an exemplary operation of the X-ray imaging system 100 for generating a corrected image I of the object 140 (see Figure 1 or 2) from the acquired projection data, in particular the acquired projection images. Preferably, the projection images comprise projection data, which correspond to an angular illumination range of each image element, i.e. of each pixel or voxel, of at least 360°. However, reconstruction of a reduced projection data set providing, for example, projection data corresponding to an angular illumination range of each image element of at least 180°is also contemplated. The X-ray detector 160 provides the acquired projection images upon detection of X-rays which have passed through the object 140 and the foreign object 190, 200 to the reconstruction processing means 170, wherein the X-rays are emitted by the radiation source 150. Based on the acquired projection images, the reconstruction processing means 170 determines the presence of the foreign object 190, 200, e.g. by use of the classification means or, in general, by a feature extraction and/or recognition algorithm, or the like. Upon determining the presence it further determines an identifier of the foreign object 190, 200 by use of the classification means of the AI-module 174, which may reveal a shape, material property, density, or the like, of the foreign object 190, 200. Based on the unique identifier, the reconstruction processing means 170 obtains a three-dimensional profile of the foreign object 190, 200 matching the unique identifier by downloading from the foreign object database 182 the server-based computing device 180. Based on the three-dimensional profile that comprises in particular geometric data, such as the shape, dimension, or the like, material data, such as the density, the density distribution, the material properties, or the like, and at least a subset of the acquired projection images, the reconstruction processing means 170 determines, e.g. by a position determining module, the position and/or orientation of the imaged foreign object 190, 200. For this, the reconstruction processing means 170 uses a few orthogonal images of the acquired projection images, a low-resolution three-dimensional image reconstructed by the reconstruction processing means 170 based on the acquired projection images, or the like. At least based on the position and/or the orientation of the imaged foreign object 190, 200, the reconstruction means 170 at least reduces an impact of the foreign object 190, 200 on the reconstructed image quality by subtracting an artefact from the reconstructed image, either during or after the final reconstruction of the image. The reconstructed and corrected image of the object 140, in which artefacts caused by the foreign object 190, 200 are reduced or from which the artefacts are substantially removed, is detonated by reference sign I in Figure 3.

Figure 4 shows a schematic block diagram of another exemplary operation of the X-ray imaging system 100 for generating a corrected image I of the object 140 (see Figure 1 or 2) from the acquired projection data, in particular the acquired projection images. In order to avoid repetition, mainly the differences to the operation according to Figure 3 will be described below. In addition to the detection of the foreign object 190, 200 (or its identifier), data obtained by the further detection means 210 is used to detect and/or identify the foreign object 190, 200. These additional data may be based on a read-out of a computer-readable identifier as the above-mentioned barcode, QR code, NFC tag, RFID tag, or the like. In addition to the position and/or orientation determination of the foreign object 190, 200 based on the image data, which comprise the image of the object 140 and the foreign object 190, 200, the further detection means 210, preferably in another configuration (not shown), is used to acquire the 6DoF position and/orientation base on EM tracking, radar or light or ultrasound triangulation, or the like. Apart from these differences, again, the reconstruction processing means 170 generates the reconstructed and corrected image I of the object 140 as explained above.

It is noted that the exemplary operations described with reference to Figures 3 and 4 may also be combined with each other.

Figure 5 shows a schematically images of the object 140 to be imaged at several points during the reconstruction and/or correction process using the reconstruction processing means 170. As indicated on the left side of Figure 5, the X-ray detector 160 provides the acquired projection images, which only for better illustration are indicated as being already reconstructed. As indicated, the acquired projection images comprise artefacts 192, 202 caused by the foreign objects 190, 200. The artefact 202 is a streak artefact due to the high density of the metal implant which is represented by the foreign object 200. As explained above, based on the acquired projection images, the reconstruction processing means 170 determines the presence of the foreign object 190, 200, e.g. by use of the classification means or, in general, by the feature extraction and/or recognition algorithm, or the like. Upon determining the presence it further determines the identifier of the foreign object 190, 200 by use of the classification means of the AI-module 174, which may reveal a shape, material property, density, or the like, of the foreign object 190, 200. Based on the unique identifier, the reconstruction processing means 170 obtains the three-dimensional profile of the foreign object 190, 200 matching the unique identifier by downloading from the foreign object database 182 the server-based computing device 180. Based on the three-dimensional profile, the reconstruction processing means 170 determines, e.g. by the position determining module, the position and/or orientation of the imaged foreign object 190, 200. At least based on the position and/or the orientation of the imaged foreign object 190, 200, the reconstruction means 170 at least reduces an impact of the foreign object 190, 200 on the reconstructed image quality by subtracting an artefact from the reconstructed image, either during or after the final reconstruction of the image. As a result, the reconstructed and corrected image I of the object 140 (see also Figure 1 or 2), in which artefacts caused by the foreign object 190, 200 are reduced or from which the artefacts are substantially removed, is obtained.

Figure 6 shows a flow chart of a method of imaging an object by the X-ray imaging system 100. In a step S1, presence of the foreign object 190, 200 located in the object 140 or located between a surface of the object 140 and the X-Ray radiation source 150 and/or the X-ray detector 160 is determined. In a step S2, the three-dimensional profile of the determined foreign object 190, 200 is obtained from the server-based foreign object database 182. In a step S3, the image I of the object 140 is reconstructed by acquiring the plurality of projection images of the object 140 from the X-ray detector 160 and reducing impact on image quality of an artefact 192, 202that can be caused by the foreign object in the image of the object 140 based on at least the obtained three-dimensional profile of the foreign object 190, 200.

In an optional step S4 (not shown), the three-dimensional profile is obtained from at least a first scan on a first radiation dosage level and a second scan on a second radiation dosage level different to the first radiation dosage level of the X-ray imaging system. The dosage level may comprise a different intensity and/or a different electromagnetic spectrum.

In an optional step S5 (not shown), a photon counting bin based scatter profile of the foreign object 190, 200 is determined before reconstructing the image I.

In an optional step S6 (not shown), a phase contrast information of the foreign object 190, 200 is determined before reconstructing the image I.

In an optional step S6 (not shown), determining the presence of the foreign object 190, 200 is performed during a scout scan provided with a first radiation dose, and acquiring the image of the object is performed by an examination scan provided with a second radiation dose higher than the first radiation dose.

In an optional step S7 (not shown), the three-dimensional profile is at least partly obtained by additive manufacturing a model of the determined foreign object 190, 200 and scanning the same by means of the X-ray imaging system 100.

It should to be noted that embodiments of the invention are described with reference to different subject-matters. In particular, some embodiments are described with reference to method-type claims, whereas other embodiments are described with reference to device-type claims. However, a person skilled in the art will gather from the above, and the following description that, unless otherwise notified, in addition to any combination of features belonging to one type of subject-matter, also other combinations between features relating to different subject-matters is considered to be disclosed with this application.

All features can be combined to provide a synergetic effect that is more than the simple summation of the features.

While the invention has been illustrated and described in detail in the drawings and foregoing description, such illustration and description are to be considered illustrative or exemplary, and not restrictive. The invention is not limited to the disclosed embodiments.

Other variations to the disclosed embodiments can be understood, and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 100: X-ray imaging system
- 110: housing
- 120: gantry
- 130: object support
- 140: object
- 150: radiation source
- 160: X-ray detector
- 170: reconstruction processing means
- 171: processor
- 172: memory
- 173: memory
- 174: artificial-intelligence-module
- 180: server-based computing device
- 181: processor, memory etc.
- 182: foreign object database
- 190: foreign object
- 191: computer-readable identifier
- 192: artefact
- 200: foreign object
- 202: artefact
- 210: detection means
- 220: further detection means

## Claims

1. X-ray imaging system (100), comprising
an X-Ray radiation source (150) configured to emit an X-ray beam towards an object (140) to be imaged,
an X-ray detector (160) configured to detect X-rays which have passed through the object,
a reconstruction processing means (170) configured to reconstruct an image of the object (140) based on the detected X-rays, wherein the reconstruction processing means (170) is further configured to:
determining presence of at least one foreign object (190, 200) located in the object (140) or located between a surface of the object (140) and the X-Ray radiation source (150) and/or the X-ray detector (160),
obtaining a three-dimensional profile of the determined foreign object (190, 200) from a server-based foreign object database (182), and
reconstructing an image (I) of the object (140) by acquiring a plurality of projection images of the object (140) from the X-ray detector (160) and reducing impact on image quality of an artefact (192, 202) that can be caused by the foreign object (190, 200) in the image of the object (140) based on at least the obtained three-dimensional profile of the foreign object (190, 200).

2. X-Ray imaging system (100) according to claim 1,
wherein, the reconstruction processing means (170) is further configured to at least partly subtract the artefact (192, 202) from the image of the object (140).

3. X-Ray imaging system (100) according to claim 1 or 2,
wherein the three-dimensional profile comprises density data and/or material data of the foreign object (190, 200).

4. X-Ray imaging system (100) according to any one of the preceding claims, wherein the reconstruction processing means comprises a classification means (174) for identifying the foreign object (190, 200) on basis of at least a feature extraction of the foreign object (190, 200) derived from the acquired image of the object including the foreign object.

5. X-Ray imaging system (100) according to any one of the preceding claims,
wherein the reconstruction processing means (170) is further configured to identify the foreign object based on a read-out of a computer-readable identifier provided along with the foreign object.

6. X-Ray imaging system (100) according to any one of the preceding claims,
wherein the reconstruction processing means (170) is further configured to determine at least a 6DoF-position of the foreign object relative to the object before reducing the impact of the foreign object.

7. X-Ray imaging system (100) according to claim 6,
wherein the reconstruction processing means (170) is further configured to determine the 6DoF-position based on a first image acquiring scan provided with a first resolution, and
based on the 6DoF-position to subtract the foreign object from a second image acquiring scan provided with a second resolution higher than the first resolution.

8. X-Ray imaging system (100) according to claim 6 or 7,
wherein the reconstruction processing means (170) is further configured to adapt scan parameters of the X-Ray imaging system based on the 6DoF-position.

9. X-Ray imaging system (100) according to any one of the preceding claims,
wherein the foreign object (190, 200) comprises an electronic device arranged between a surface of the object and the X-Ray radiation source (150) and/or the X-ray detector (160).

10. Method of imaging an object by an X-ray imaging system (100), comprising
determining presence of at least one foreign object (190, 200) located in the object or located between a surface of the object and the X-Ray radiation source (150) and/or the X-ray detector (160),
obtaining a three-dimensional profile of the determined foreign object (190, 200) from a server-based foreign object database (182), and
reconstructing an image of the object by acquiring a plurality of projection images of the object from the X-ray detector (160) and reducing impact on image quality of an artefact that can be caused by the foreign object (190, 200) in the image of the object based on at least the obtained three-dimensional profile of the foreign object.

11. Method according to claim 10,
wherein the three-dimensional profile is obtained from at least a first scan on a first radiation dosage level and a second scan on a second radiation dosage level different to the first radiation dosage level of the X-ray imaging system (100).

12. Method according to claim 10 or 11,
wherein a photon counting based scatter profile of the foreign object (190, 200) is determined before reconstructing the image.

13. Method according to any one of claims 10 to 12,
wherein a phase contrast information of the foreign object (190, 200) is determined before reconstructing the image.

14. Method according to any one of claims 10 to 13,
wherein the three-dimensional profile is at least partly obtained by additive manufacturing a model of the determined foreign object and scanning the same by means of the X-ray imaging system (100).

15. A computer program element, which, when being executed by at least one processing unit, is adapted to cause the processing unit to perform the method according to any one of claims 10 to 14.
